Europäisches Patentamt

European Patent Office

Office européen des brevets

(19)

(11) Numéro de publication : **0 189 389**
**B1**

(12) **FASCICULE DE BREVET EUROPÉEN**

(45) Date de publication du fascicule du brevet :
05.04.89

(21) Numéro de dépôt : 86870009.7

(22) Date de dépôt : 21.01.86

(51) Int. Cl.⁴ : **G 01 N 33/546,**
**G 01 N 33/555,**
**G 01 N 33/569**

(54) Procédé de dosage immunologique d'anticorps de diverses classes dans un échantillon liquide.

(30) Priorité : 24.01.85 BE 214388

(43) Date de publication de la demande :
30.07.86 Bulletin 86/31

(45) Mention de la délivrance du brevet :
05.04.89 Bulletin 89/14

(84) Etats contractants désignés :
AT BE CH DE FR GB IT LI LU NL SE

(56) Documents cités :
EP--A-- 0 019 741
EP--A-- 0 101 166
DE--A-- 2 746 101
GB--A-- 2 001 171
US--A-- 3 600 494

(73) Titulaire : **INSTITUT INTERNATIONAL DE PATHOLO-GIE CELLULAIRE ET MOLECULAIRE**
**Avenue Hippocrate 75**
**B-Woluwé-Saint-Lambert (BE)**

(72) Inventeur : **Cambiaso, Cesar L.**
**rue Frans Cloetens 13**
**B-1950 Kraainem (BE)**
Inventeur : **Masson, Pierre L.**
**Avenue Emile Vandervelde 107**
**B-1200 Bruxelles (BE)**

(74) Mandataire : **Schmitz, Yvon et al**
**Bureau Gevers S.A. rue de Livourne 7 bte 1**
**B-1050 Bruxelles (BE)**

Jouve, 18, rue St-Denis, 75001 Paris, France

## Description

La présente invention est relative à un procédé de dosage immunologique d'anticorps de diverses classes, dirigés contre un ou plusieurs antigènes déterminés, et plus particulièrement d'anticorps IgM dans un échantillon liquide contenant des anticorps IgM, IgG et éventuellement d'autres classes, telles que IgA, IgD, IgE, tel qu'un fluide biologique.

Lorsqu'une personne est infectée par une bactérie ou un virus, elle développe des anticorps généralement de diverses classes pour détruire les agents infectueux. Initialement, les anticorps sont principalement de classe IgM mais progressivement les IgG prédominent et peuvent persister pendant plusieurs années. Par conséquent, le fait de déterminer simplement si des anticorps existent est peu pratique dans le diagnostic d'une infection bien que cela puisse être utile pour déterminer si la personne présente une immunité ou une résistance à un type particulier d'infection. Si, toutefois, on détecte l'anticorps qui s'est formé le premier, c'est-à-dire IgM et que l'on estime sa concentration relative comparativement à IgG, on peut savoir si l'infection est récente ou ancienne. Ces estimations sont extrêmement importantes pendant les premiers temps de la grossesse lorsque, par exemple, diverses infections dues à des bactéries, des protozoaires ou des virus peuvent avoir atteint le fœtus.

Une difficulté en ce qui concerne cette estimation est la différence entre la concentration en IgG et IgM. En termes de concentration moléculaire, on peut s'attendre à ce qu'il y ait, par exemple, 10 fois plus d'IgG présents dans le sérum humain que d'IgM. On peut s'attendre à ce que la proportion d'anticorps IgM ou IgG, normalement de moins de 2 % des IgM ou des IgG totales, soient les mêmes pour les deux immunoglobulines.

Un grand nombre de procédés décrits dans la littérature permet de différencier les anticorps IgM des IgG [Chantler et Diment, Current status of specific IgM antibody assays (1981), 417, dans Immunoassays for the 80's, M.T.P. Press Limited, Lancaster ; Grande-Bretagne].

Tous les procédés à l'exception du procédé d'agglutination de globules rouges [Coombs et col. Lancet (1968), 2,1115] se fondent sur la liaison d'un antigène ou d'un anticorps aux parois d'un tube ou puits d'une plaque de microtitrage pour absorber sélectivement l'anticorps choisi. Les tubes ou puits sont lavés pour éliminer toute interférence provoquée par les immunoglobulines non désirées et d'autres substances susceptibles d'interférence éventuelles et l'on utilise ensuite une méthode de marquage de l'anticorps IgM (ou IgG) pour quantifier l'anticorps à mesurer [voir, par exemple, la description d'Organon Teknika, Oss, Hollande, de leur système « Toxonostika » (marque déposée) IgM et IgG « Microelisa »]. Ces procédés requièrent un grand nombre d'étapes manuelles, une séparation physique des anticorps liés de ceux qui ne sont pas liés, des lavages et de longues durées d'incubation. Une substance d'interférence majeure dans la plupart des essais est l'anticorps appelé « facteur rhumatoïde » ou « RF », qui est une IgM qui se lie à n'importe quelle IgG qui a réagi avec un antigène polyvalent ou qui a été agrégée par des procédés physiques ou chimiques, et qui apparaît dans des proportions importantes dans tous les échantillons. De plus, un grand nombre de procédés ne sont que semi-quantitatifs, c'est-à-dire qu'on ne peut pas exprimer les résultats en quantités pondérales.

L'objet de la présente invention consiste, par conséquent, à palier les inconvénients susmentionnés des procédés de dosage connus, et à réaliser un procédé de dosage immunologique d'anticorps IgM dans un échantillon liquide contenant des anticorps IgM, IgG et éventuellement d'autres classes, telles que IgA, IgD, IgE, dirigés contre au moins un antigène déterminé, tel qu'un fluide biologique, extrêmement rapide, ne requérant pas plus de 60 minutes et qui est quantitatif. Le procédé de l'invention ne requiert aucune séparation physique des anticorps IgM des anticorps IgG ou du sérum ou du fluide corporel dans lequel on effectue la mesure. De plus, la même technique permet de mesurer, par exemple, la quantité d'anticorps non-IgM dans un échantillon liquide contenant des anticorps IgM et d'autres classes. A l'inverse des autres techniques, le procédé de la présente invention peut également être totalement automatisé.

A cet effet, suivant l'invention, on fait réagir l'échantillon liquide contenant les anticorps IgM, IgG et éventuellement d'autres classes, telles que IgA, IgD, IgE, dirigés contre au moins un antigène déterminé, avec des anticorps dirigés contre les IgG et des anticorps dirigés contre les IgA dans le cas où l'échantillon contient des anticorps IgA dirigés contre ce même antigène (les anticorps IgD et IgE sont généralement en quantité négligeable), on ajoute au produit de réaction ainsi obtenu ce même antigène lié à des particules finement divisées, telles que, par exemple, des particules de latex ou des globules rouges, et un antigène libre du même type que l'antigène lié, et on détermine le degré d'agglutination ou de non agglutination de ces particules finement divisées, la quantité d'anticorps IgM étant inversement proportionnelle à la quantité de ces particules finement divisées non agglutinées.

Suivant une forme de réalisation particulière du procédé de l'invention, après avoir ajouté les anticorps dirigés contre les IgG et éventuellement les anticorps dirigés contre les IgA et incubé la solution ainsi obtenue, on ajoute à celle-ci des immunoglobulines IgG agrégées pour neutraliser l'effet provoqué par le facteur rhumatoïde et éviter ainsi un renforcement de l'agglutination par les anticorps IgG qui n'auraient pas été complètement neutralisés par les anticorps anti-IgG et par l'antigène libre.

Suivant une autre forme de réalisation particulière de l'invention, on détermine la quantité de particules finement divisées agglutinées ou non agglutinées dans la suspension résultante en mesurant l'effet néphélémétrique ou turbidimétrique de celle-ci, ou par comptage de ces particules agglutinées ou non agglutinées.

L'invention se rapporte également à un procédé de dosage immunologique d'anticorps IgG + IgA dans un échantillon liquide contenant des anticorps IgM, IgG et IgA, tel qu'un fluide biologique, dans lequel on détermine tout d'abord la quantité d'anticorps IgM dans l'échantillon liquide par le procédé susmentionné, et dans lequel on soustrait ensuite la quantité d'anticorps IgM ainsi obtenue de la quantité d'anticorps totale IgM + IgG + IgA de manière à obtenir la quantité d'anticorps IgG + IgA, la quantité d'activité d'anticorps totale étant basée, par exemple, sur le principe d'agglutination par les anticorps de latex recouvert de l'antigène et de comptage des particules non agglutinées, tel qu'il a été décrit dans un grand nombre de publications [par exemple Masson et col., Particle Counting Immunoassay (PACIA) dans Methods in Enzymology (1981) 74 ; 106 Academic Press].

La caractéristique du procédé de la présente invention est de limiter l'activité agglutinante aux anticorps IgM et d'utiliser, par conséquent, la technique d'agglutination bien établie pour estimer de façon précise la présence de ces anticorps IgM en présence des autres anticorps, c'est-à-dire IgG et éventuellement IgA. Ainsi que cela est bien connu, lorsque du latex « Lx » est recouvert d'un antigène « Ag » (provenant, par exemple, de la bactérie ou du virus provoquant la maladie donnant lieu à l'anticorps) et que l'on fait passer le LxAg dans un compteur de cellules, il donnera un pic proportionnel à la concentration en particules de LxAg isolées. Lorsque des anticorps de la classe IgM (IgMac) sont amenés en contact avec LxAg, ces anticorps entraîneront une certaine agglutination de LxAg, en diminuant le nombre de particules libres LxAg et en diminuant, par conséquent, leur concentration. Cette diminution est directement proportionnelle à la quantité d'anticorps totale (IgGAc, IgMAc et éventuellement IgAAc) présente dans l'échantillon mesuré.

Il semblait logique que l'on puisse éliminer la réaction par les IgGAc en ajoutant une substance qui agrégerait les IgGAc et, par conséquent, qui les éliminerait de la réaction avec le latex, en laissant simplement la réaction avec les IgMAc, ainsi que cela a été décrit par Gipsen et coll. [Clin. Exp. Immunology (1975) 22, 321] ou Schmitz et coll. (J. Clin. Microbiol. (1975), 1, 132). Dans le cas où l'échantillon liquide examiné contient également des anticorps IgA (IgAAc), ceux-ci pourraient également être éliminés de la réaction avec le latex en leur ajoutant une substance qui les agrégerait. On choisit, suivant la présente invention, comme réactif spécifique pour inactiver totalement les IgG, un antisérum provenant de chèvre, dirigé vers ce que l'on appelle la région Fc de l'IgG humaine (anti-Fc) et connu pour agréger sélectivement les anticorps IgG humains. Lorsque la totalité des anticorps IgM est inactivée par traitement avec du dithiothréitol (DDT), qui n'active pas les anticorps IgG, on obtient encore une agglutination de LxAg mais nettement moindre. Si l'on ajoute à cette suspension des anticorps anti-Fc pour agréger les anticorps IgG, on ne remarque plus aucune agglutination de LxAg. On suppose, par conséquent, que l'agglutination du latex par anticorps IgG est inhibée par les anticorps dirigés contre les IgG. D'une façon inattendue, le LxAg s'agglutine plus faiblement qu'attendu lorsque l'on ajoute des quantités connues, relativement élevées, d'IgMac (sans traitement au DDT) aux IgGAC qui ont été agrégés par des anticorps anti-Fc ; ce qui montre que l'antigène Ag sur le latex est vraisemblablement masqué par les complexes IgGAc-anti-Fc. Pour bloquer la réaction entre les anticorps IgG et l'antigène de LxAg, on ajoute alors de l'antigène libre « Ag » à la suspension. On aurait pu supposer qu'un tel antigène bloquerait également les anticorps IgM susceptibles d'agglutiner le latex. D'une façon surprenante, ce bloquage ne se réalise pas dans le cas où la concentration d'antigène libre n'est pas excessive. En utilisant de l'antigène libre Ag avec des anticorps anti-Fc pour agréger la totalité des anticorps IgG, la concentration en particules libres est apparemment la même que dans le cas où l'agglutination est due à des anticorps IgM seuls. Cette constatation, bien que surprenante dans son effet, est conforme aux caractéristiques connues des deux anticorps. Les IgMc ont habituellement une affinité plus faible que les IgGAc. Toutefois, du fait de leur valence multiple (10 valences), les IgMAc réagissent préférentiellement avec l'antigène porteur de déterminants antigéniques répétés. Le couplage de l'Ag au latex rend l'Ag répétitif et par conséquent fortement réactif avec les IgMAc. Une faible concentration en antigène libre peut par conséquent saturer la capacité totale de liaison des anticorps IgG tandis qu'une concentration beaucoup plus élevée en Ag libre, pour autant qu'il ne contienne pas des déterminants antigéniques répétitifs, est requise pour saturer des anticorps IgM.

Le procédé de la présente invention permet, par conséquent, de mesurer l'activité en anticorps IgM en utilisant, par exemple, des anticorps anti-Fc dirigés contre les IgG et, bien entendu, des anticorps dirigés contre les IgA dans le cas où l'échantillon liquide à examiner contient des anticorps IgA, les anticorps IgD et IgE se trouvant généralement, ainsi qu'on l'a déjà précisé précédemment, en quantité négligeable, et un antigène libre, les anticorps IgG venant en fait se combiner à l'antigène libre, cette combinaison avec l'antigène venant ainsi compléter l'inactivation des anticorps IgG et éventuellement des anticorps IgA.

Le procédé de l'invention permet également, ainsi qu'on l'a déjà précisé précédemment, de doser les anticorps IgG + IgA dans un échantillon liquide contenant des anticorps IgM, IgG et IgA, en déterminant tout d'abord la quantité d'anticorps IgM dans l'échantillon liquide à examiner par le procédé de l'invention, qui vient d'être décrit, et en soustrayant ensuite la quantité d'anticorps IgM ainsi obtenue de la quantité d'anticorps totale IgM + IgG + IgA de manière à obtenir la quantité d'anticorps IgG + IgA. Pour obtenir la quantité d'anticorps IgG, il suffira bien entendu d'ajouter au mélange de réaction des anticorps anti-IgA et, de la même manière, pour obtenir la quantité d'anticorps IgA, il suffira d'ajouter au mélange de réaction des anticorps anti-IgG.

Un problème qui a amené un grand nombre de procédés de la technique antérieure à donner des résultats faussement positifs, consiste en l'interférence que provoque le facteur rhumatoïde (FR) qui est un anticorps IgM qui se lie à la plupart des complexes IgGAc-Ag. Suivant l'invention, on a neutralisé l'effet de ce facteur rhumatoïde par addition d'IgG agrégées. L'efficacité de cette étape a été testée par addition d'un sérum contenant une concentration très élevée en facteur rhumatoïde mais dépourvu d'IgMAc, après l'addition des anticorps dirigés contre les IgG et éventuellement des anticorps dirigés contre les IgA et incubation du mélange ainsi obtenu. En aucun cas, un résultat sensiblement différent n'a été obtenu.

On notera qu'un test d'agglutination de latex pour la détermination spécifique des IgMAc a récemment été décrit [J.S. Remington et coll., Detection of Immunoglobulin M Antibodies with antigen tagged latex particles in an immunosor-bent assay, J. Clin. Microbiology (1983), volume 17, n° 5, page 939]. Comme avec presque tous les essais précédents, les anticorps dirigés contre les anticorps IgM humains sont liés aux parois du puits d'une plaque de microtitrage. Lorsque l'on ajoute du sérum, une certaine proportion de la totalité des IgM adhère aux parois, y compris les anticorps IgM. Une fois cette réaction réalisée, on ajoute des particules de latex recouvertes d'antigène. Après plusieurs heures, on note la quantité de latex lié à la microplaque et l'on compare celle-ci aux résultats obtenus par des concentrations connues en anticorps IgM et on effectue ainsi une estimation de la quantité d'anticorps présents. Les limitations de ce procédé résident dans le fait que l'estimation n'est que semi-quantitative. Le procédé ne tient compte que des anticorps IgM, comporte de longues périodes d'incubation, requiert un lavage pour séparer les substances interférentes et demande beaucoup de travail. De plus, lorsque le patient a un taux d'immunoglobulines IgM élevé, la proportion d'IgMAc liés diminuera, en réduisant ainsi la sensibilité avec le danger que l'on puisse rater des résultats positifs.

On notera, à cet effet, que les périodes d'incubation utilisées dans le procédé de la présente invention sont de courte durée. C'est ainsi qu'après avoir ajouté les anticorps dirigés contre les IgG et éventuellement les anticorps dirigés contre les IgA, on incube la solution ainsi obtenue sous agitation à une température d'environ 37 °C pendant une durée de l'ordre de 1 à 15 minutes. D'autre part, après avoir ajouté l'antigène lié à des particules finement divisées et l'antigène libre, on incube également la suspension ainsi obtenue sous agitation à une température d'environ 37 °C pendant une durée de l'ordre de 1 à 15 minutes, une incubation similaire s'avérant nécessaire après l'addition éventuelle des IgG agrégées utilisées pour neutraliser l'effet provoqué par le facteur rhumatoïde.

Bien que le procédé de l'invention puisse être appliqué à n'importe quelle infection, par exemple du type toxoplasmose, cytomégalovirus, herpès, rubéole, etc., on décrira deux exemples de réalisation se rapportant à la détermination d'anticorps IgG et IgM dirigés, d'une part, contre Brucella abortus et, d'autre part, contre Toxoplasma gondii. L'antigène de Brucella est le lipopolysaccharide (LPS).

Exemple 1

Préparation de LPS

Du LPS de Brucella est extrait au moyen du procédé à l'eau-phénol de Westphal et coll. [Methods in Carbohydrate Chemistry, volume 5 (1965), page 83, Academic Press, N.Y.]. La phase phénolique est ensuite précipitée par trois volumes de « réactif méthanolique » et traitée par du diméthylsulfoxyde dans du NaI (5 cycles), comme décrit par Moreno et coll., [J. Bacteriology (1979), 361, 138, n° 2]. On dialyse ensuite le LPS contre de l'eau désionisée et on le lyophilise.

Préparation du latex-antigène (LxLPS)

A 50 μg de LPS dissous dans 0,4 ml de GBS dilué 5 fois (dGBS) (GBS : solution saline tamponnée à base de glycine de pH 9,2), on ajoute 50 μl de latex (10 % en poids/volume) (K109 - Rhône-Poulenc, Courbevoie, France) et on soumet le mélange aux ultrasons pendant 1 minute à 30 watts (appareil à ultrasons modèle B12, Branson, Danbury, CT, 06810). Après 10 minutes, on ajoute 100 μg d'albumine de sérum humain (HSA, Behringwerke, Marburg/Lahn, FRG) dans du GBS, et on soumet à nouveau la suspension aux ultrasons. Après 20 minutes d'incubation à la température ambiante et centrifugation pendant 10 minutes à 10 000 tours/minute dans un rotor SS 34 d'une centrifugeuse Sorvall R5 CB, le latex est remis en suspension dans 1 ml de solution aqueuse de dodécyl sulfate de sodium, soumis aux ultrasons et incubé pendant 1 heure à 37 °C. On lave ensuite le latex deux fois avec 1 ml de dGBS et on le remet en suspension dans 1 ml d'EDTA (éthylène diaminetétraacétate)-GBS-BSA (albumine de sérum bovin). On constate que le latex stocké congelé à — 20 °C ou lyophilisé est stable pendant au moins 6 mois. On soumet aux ultrasons pendant 15 secondes à 30 watts du LxLPS qui a été dégelé ou resuspendu après lyophilisation. Avant utilisation, on dilue le LxLPS 100 fois dans du EDTA-GBS-BSA.

Anticorps anti-Fc

On prépare le fragment Fc d'IgG humaine par digestion à la papaïne d'après le procédé de Cerottini et coll., [J. Immunology (1968) 101, page 433]. On injecte 100 µg de Fc humain dans de l'adjuvant de Freund complet par voie intradermique chez une chèvre à des intervalles de 3 semaines. On recueille le sérum 15 jours après l'injection finale, on extrait les immunoglobulines et on les utilise diluées dans du GBS (1 mg/ml) après avoir vérifié leur spécificité anti-Fc.

Procédé

On ajoute 25 µl d'anti-Fc à 50 µl de sérum humain dilué 50 fois dans du GBS. On incube le mélange à 37 °C pendant 2 minutes tout en agitant. On ajoute ensuite 25 µl d'immunoglobulines IgG humaines agrégées à une concentration de 25 mg/ml et on incube à nouveau le mélange pendant 10 minutes à 37 °C. Finalement, on ajoute à ce mélange 25 µl de LxLPS mélangé à de l'antigène libre (LPS) à une concentration de 5 µg/ml et on poursuit l'incubation pendant 10 minutes supplémentaires à 37 °C. On arrête la réaction par l'addition de 2 ml de GBS et on fait ensuite passer le mélange résultant dans un compteur optique de cellules où l'on détermine la concentration de LxLPS non agglutiné. La concentration en anticorps IgM est inversement proportionnelle à la concentration de particules de latex libres. On pourrait évidemment également mesurer directement la quantité de LxLPS agglutiné ou utiliser pour le comptage une autre méthode qu'une méthode optique, par exemple le comptage de particules par résistance électrique. Pour éviter les erreurs dues à une concentration excessive en anticorps IgM, on répète le processus avec du sérum dilué 100 fois et 200 fois.

Suivant une variante, on peut mesurer l'agglutination du latex par turbidimétrie dans le cas où les concentrations d'anticorps IgM et IgG sont relativement élevées. Les mêmes quantités de sérum humain dans le GBS et d'anti-Fc sont incubées pendant 2 minutes à 37 °C avec agitation. On ajoute ensuite 25 µl d'IgG humaines agrégées à la concentration susmentionnée et on laisse le mélange incuber pendant 1 heure, après quoi on ajoute 30 µl de LxLPS à une concentration de 0,02 %. On agite rapidement le mélange et on mesure la turbidité à 620 nm et à 405 nm, la longueur d'onde de 620 nm constituant la ligne de base. On laisse ensuite le mélange incuber pendant 2 heures, après quoi on mesure l'effet turbidimétrique de celui-ci une seconde fois aux deux longueurs d'onde susmentionnées. La diminution de la différence entre les mesures prises initialement à 620 nm et 405 nm et celles prises après l'incubation de 2 heures est inversement proportionnelle aux particules libres restantes. On pourrait également déterminer la quantité de particules finement divisées agglutinées ou non agglutinées dans la suspension résultante par d'autres mesures de densité optique que la turbidimétrie, telles que la néphélémétrie.

Pour ce qui est du dosage de l'activité d'anticorps totale, c'est-à-dire de l'activité des anticorps IgM + IgG (le sérum cité en exemple ci-dessus ne contenant pas d'anticorps IgA) on utilise du tampon GBS seul à la place des anticorps anti-Fc et on utilise du LxLPS à la place du LxLPS + LPS.

Pour déterminer la concentration en anticorps IgG seuls, on utilise le même procédé que pour la détermination de l'activité d'anticorps totale, à l'exception que l'on détruit les anticorps IgM de l'échantillon en traitant préalablement l'échantillon par du dithiothréitol (DTT). A 50 µl de sérum avant la dilution avec le GBS, on ajoute 170 µl d'EDTA à une concentration 50 millimolaire dans du GBS de pH 9,2. On ajoute à ce mélange 15 µl de DTT dans de l'eau à une concentration de 10 mg/ml et on incube ensuite le mélange entier à 37 °C pendant 30 minutes, après quoi on arrête la réaction du DTT par l'addition de 15 µl d'une solution de peroxyde d'hydrogène à 0,2 %. On procède ensuite comme décrit ci-dessus et on dilue la solution obtenue à 1/10, 1/20 et 1/40 pour effectuer les mesures susmentionnées.

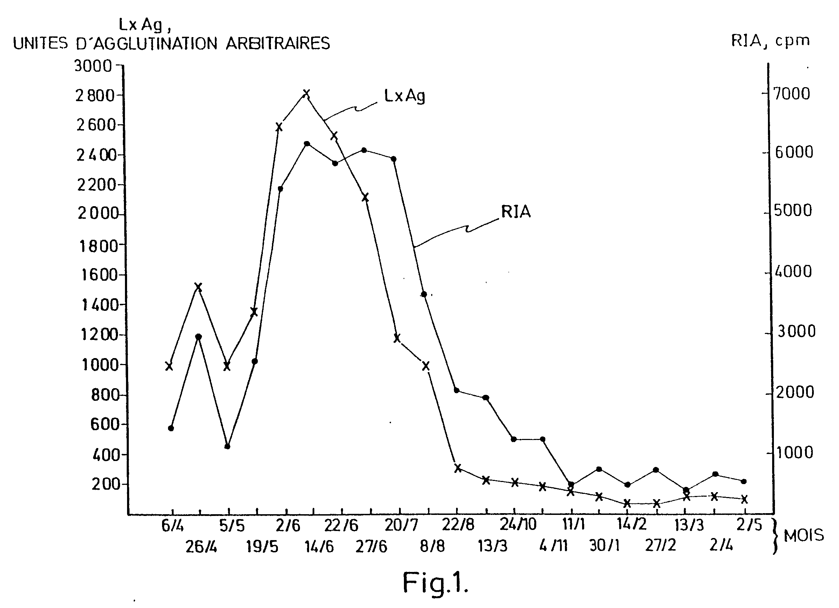

La figure 1 annexée montre les concentrations en anticorps IgM du même sérum d'un patient qui a contracté une infection à Brucella abortus, obtenues par un procédé de radioimmunoessai (RIA) (en coups par minute) et par le procédé de l'invention (en unités d'agglutination arbitraires) sur une période d'un an.

Comme on pourra le noter, les deux courbes sont similaires, ce qui démontre que le procédé de dosage de l'invention est fiable.

Exemple 2

Mesure d'anticorps de toxoplasmose IgG et IgM

Préparation d'antigène de toxoplasmose (toxoantigène)

On prépare l'antigène de toxoplasmose au départ de l'exsudat péritonéal de souris infectées trois jours avant par des trophozoïtes de T. gondii. La cavité péritonéale est lavée deux à trois fois avec 3-5 ml de solution saline physiologique stérile et les lavages sont ensuite rassemblés. A 1 ml de cette suspension, on ajoute 0,05 ml de l'antibiotique Pentrexyl et 10 unités d'héparine, et on laisse le tout au repos pendant 1/2 heure à la température ambiante dans des tubes pourvus de fonds coniques. Les tubes sont ensuite centrifugés pendant 4 minutes à 600 tours/minute, le surnageant est enlevé, le culot remis en suspension dans une solution saline et à nouveau centrifugé pendant 1/2 heure à 4 000 tours/minute à 4 °C. Après avoir ajouté au culot 2 ml d'NH₄Cl 1 M pour hémolyser les globules rouges, on centrifuge à nouveau à 4 000 tours/minute à 4 °C, on lave le culot trois fois avec une solution saline, et on centrifuge à nouveau pendant 1/2 heure à 4 000 tours/minute à 4 °C.

On remet en suspension le culot dans 2 ml d'eau distillée et ensuite, de façon répétée, on le congèle avec un mélange de carboglace et d'acétone et on le dégèle, en soumettant chaque fois la solution à une opération de mélange aux ultrasons. On centrifuge pendant 10 minutes à 3 000 tours/minute. On sépare le surnageant et on le fait passer dans une colonne d'Ultrogel 3-4 équilibrée par du PBS-NaCl (1 M). Les fractions sont testées contre du latex-Fc(ab')$_2$ anti-toxoplasmose et les fractions qui agglutinent le latex, sont rassemblées, concentrées et stockées à — 20 °C.

Préparation du latex-toxoantigène (Lxtoxo)

A 250 µl de latex carboxylé (10 % en poids/volume) (Lx), on ajoute 2 ml de solution saline tamponnée au barbital (BBS) (0,02 M, pH de 8,1), et on centrifuge pendant 10 minutes à 10 000 tours/minute. On sépare le surnageant, on ajoute 250 µl de BBS et on refroidit à 4 °C. On ajoute 250 µl de BBS contenant 25 mg de carbodiimide (CDI) pour donner un mélange de 10 mg de CDI/100 µl de Lx, cette solution fraîchement préparée, étant maintenue à une température de 4 °C. On incube pendant 1 heure avec une agitation tourbillonnaire à 40 °C.

On centrifuge à 4 °C pendant 10 minutes à 10 000 tours/minute et on ajoute 0,5 ml de BBS froid. Pendant que le mélange est dans un bain de glace, on le soumet à une vibration par ultrasons, et on l'ajoute à la solution de toxoantigène (1,5 mg) dans du BBS à 40 °C (volume ± 0,5 ml).

On incube à 4 °C pendant 2-4 heures sous une agitation tourbillonnaire ou pendant 16 heures sur un cylindre.

On lave la suspension de latex trois fois avec du GBS à 1 % et on la soumet à une opération de mélange avec ultrasons après chaque centrifugation.

Conservation : dilué à 1/20, congelé ou lyophilisé

Anticorps anti-Fc

On prépare le fragment Fc d'IgG humaine par digestion à la papaïne d'après le procédé de Cerottini et coll., [J. Immunology (1968) 101, page 433]. On injecte 100 µg de Fc humain dans de l'adjuvant de Freund complet par voie intradermique chez une chèvre à des intervalles de trois semaines. On recueille le sérum 15 jours après l'injection finale, on extrait les immunoglobulines et on les utilise diluées dans une solution saline à 0,9 % (12 mg/ml) après avoir vérifié leur spécificité anti-Fc.

Procédé

A des échantillons de 50 µl de sérum humain, dilué 50 fois (ou 25 fois) dans du GbS/1 % de BSA, on ajoute 25 µl de solution d'anti-Fc (12 mg/ml dans une solution saline à 0,9 %) et on laisse incuber pendant 10 minutes à 37 °C. A ce mélange, on ajoute ensuite 25 µl d'une solution d'IgG humaines agrégées (par chauffage d'IgG pendant 30 minutes à 63 °C) à une concentration de 10 mg/ml dans une solution saline à 0,9 % et on incube à nouveau à 37 °C pendant 10 minutes. Finalement, on ajoute 50 µl de Lxtoxo contenant de l'antigène de toxoplasmose libre à une concentration de 0,1 µg/ml. On poursuit l'incubation pendant une nouvelle période de 30 minutes à 37 °C et on arrête la réaction par l'addition de 2,0 ml de GBS. On fait passer le mélange résultant dans un compteur optique de cellules où seules les particules de latex non agglutinées sont comptées, leur concentration étant inversement proportionnelle à la concentration en anticorps IgM.

Suivant une variante, on peut mesurer la concentration de particules de latex libres par turbidimétrie dans le cas où les concentrations d'anticorps IgM et IgG sont relativement élevées. Les mêmes quantités de sérum humain dans le GBS et d'anti-Fc sont incubées pendant 2 minutes à 37 °C avec agitation tourbillonnaire. On ajoute ensuite 25 µl d'IgG humaines agrégées à la concentration susmentionnée et on laisse le mélange incuber pendant 1 heure, après quoi on ajoute 30 µl de Lxtoxo à une concentration de 0,02 %. On agite rapidement le mélange et on mesure la turbidité à 620 nm et à 405 nm, la longueur d'onde de 620 nm constituant la ligne de base. On laisse ensuite le mélange incuber pendant 2 heures, après quoi on mesure l'effet turbidimétrique de celui-ci une seconde fois aux deux longueurs d'onde susmentionnées. La diminution de la différence entre les mesures prises initialement à 620 nm et 405 nm et celles finalement notées est inversement proportionnelle aux particules libres restantes.

Pour ce qui est du dosage de l'activité des anticorps totaux, on utilise du tampon GBS seul à la place des anticorps anti-Fc et du Lxtoxo sans toxoantigène libre.

Pour déterminer la concentration en anticorps IgG seuls, on utilise le même procédé que pour la détermination de l'activité d'anticorps totaux, à l'exception que l'on détruit les anticorps IgM de l'échantillon en traitant préalablement l'échantillon par du dithiothréitol (DTT). A 50 µl de sérum avant la dilution avec le GBS, on ajoute 170 µl d'EDTA à une concentration 50 millimolaire dans du GBS de pH 9,2. On ajoute à ce mélange 15 µl de DTT dans de l'eau à une concentration de 10 mg/ml, et on incube ensuite le mélange entier à 37 °C pendant 30 minutes, après quoi on arrête la réaction du DTT par l'addition de 15 µl d'une solution de peroxyde d'hydrogène à 0,2 %. On procède ensuite comme décrit ci-dessus, et on dilue la solution obtenue à 1/10, 1/20, 1/40 pour effectuer les mesures susmentionnées.

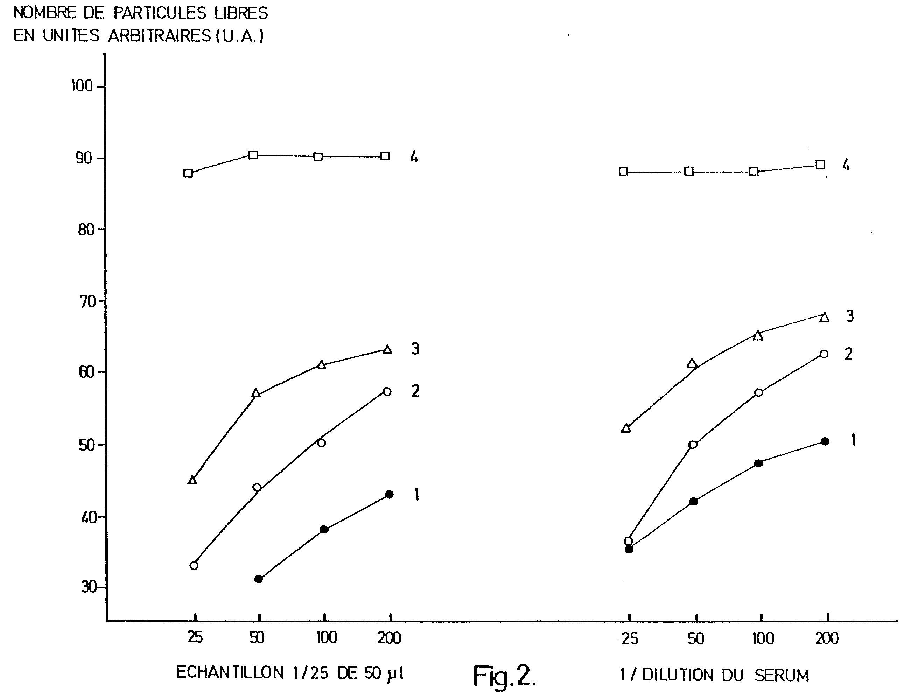

La figure 2 montre les concentrations relatives dans deux sérums (dilution à 1/25) en anticorps IgG et IgM, c'est-à-dire en anticorps totaux (courbe 1), en anticorps IgG (courbe 2), en anticorps IgM (courbe 3) et finalement avec l'activité d'anticorps totale inhibée par l'addition de DTT (contre les anticorps IgM) et d'anticorps anti-Fc (contre les anticorps IgG) (courbe 4) en fonction de la dilution des sérums.

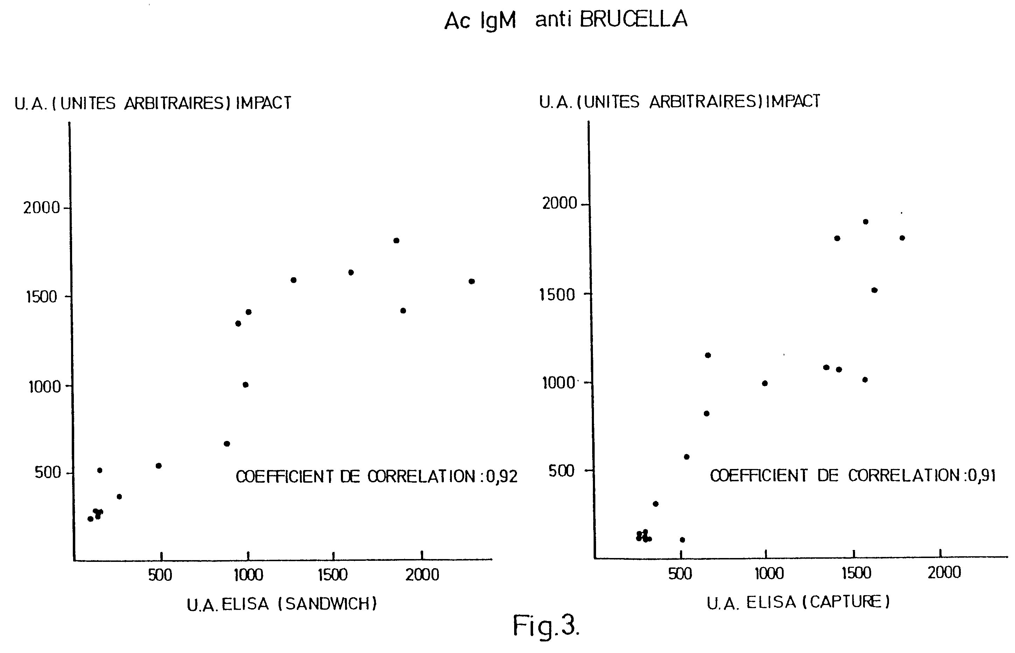

La figure 3 montre la relation entre les résultats de la détermination en anticorps IgM contre Brucella par le système IMPACT (marque déposée) et les résultats obtenus par ELISA appliqué soit selon le mode « sandwich » ou le mode « capture ». Les coefficients de corrélation étaient de 0,92 pour la première comparaison et de 0,91 pour la seconde.

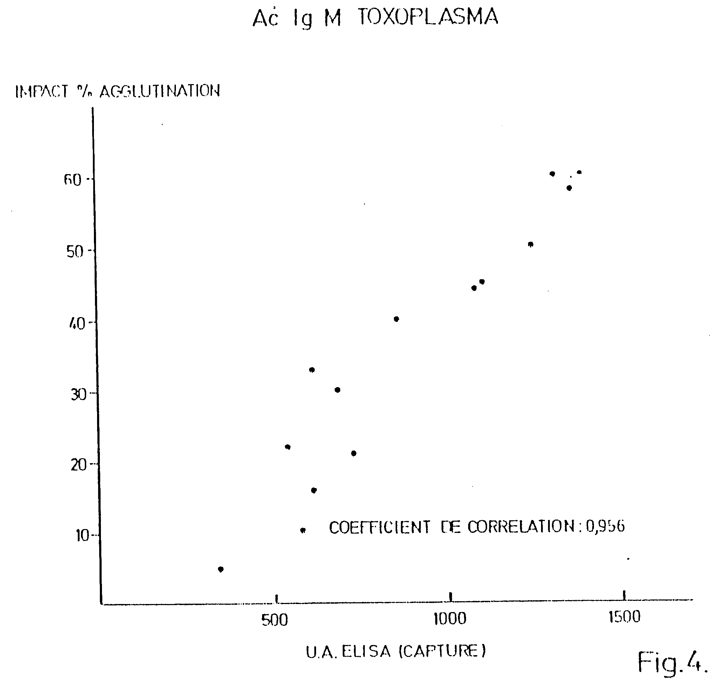

La figure 4 montre la relation entre les techniques IMPACT (marque déposée) et ELISA (capture) pour la détermination d'anticorps IgM contre la toxoplasmose. Le coefficient de corrélation est de 0,96.

Ainsi qu'on l'a déjà précisé précédemment, on pourrait bien entendu utiliser comme particules finement divisées, à la place des particules de latex, des globules rouges.

**Revendications**

1. Procédé de dosage immunologique d'anticorps IgM, dirigés contre au moins un antigène déterminé, dans un échantillon liquide contenant au moins des anticorps IgM et IgG, tel qu'un fluide biologique, caractérisé en ce qu'il comprend la mise en réaction dudit échantillon liquide contenant les anticorps IgM, IgG et éventuellement des anticorps IgA (dirigés contre ce même antigène), d'anticorps dirigés contre les IgG et d'anticorps dirigés contre les IgA dans le cas où l'échantillon susdit contient des anticorps IgA, l'addition au produit de réaction ainsi obtenu de ce même antigène lié à des particules finement divisées et d'un antigène libre du même type que l'antigène lié et la détermination du degré d'agglutination ou de non agglutination de ces particules finement divisées, la quantité d'anticorps IgM étant inversement proportionnelle à la quantité de ces particules finement divisées non agglutinées.

2. Procédé suivant la revendication 1, caractérisé en ce qu'après avoir ajouté les anticorps dirigés contre les IgG et éventuellement les anticorps dirigés contre les IgA, on incube la solution ainsi obtenue sous agitation à une température d'environ 37 °C pendant une durée de l'ordre de 1 à 15 minutes.

3. Procédé suivant l'une ou l'autre des revendications 1 et 2, caractérisé en ce qu'après avoir ajouté l'antigène susmentionné lié à des particules finement divisées et l'antigène libre, on incube la suspension ainsi obtenue sous agitation à une température d'environ 37 °C pendant une durée de l'ordre de 1 à 15 minutes.

4. Procédé suivant l'une ou l'autre des revendications 2 et 3, caractérisé en ce qu'après avoir ajouté les anticorps dirigés contre les IgG et éventuellement les anticorps dirigés contre les IgA et incubé la solution ainsi obtenue, on ajoute à celle-ci des IgG agrégées pour neutraliser l'effet provoqué par le facteur rhumatoïde.

5. Procédé suivant la revendication 4, caractérisé en ce qu'après avoir ajouté les IgG agrégées à la solution incubée ainsi obtenue, on incube à nouveau celle-ci sous agitation à une température d'environ 37 °C pendant une durée de l'ordre de 1 à 15 minutes.

6. Procédé suivant l'une quelconque des revendications 1 à 5, caractérisé en ce que l'on détermine la quantité de particules finement divisées agglutinées ou non agglutinées dans la suspension résultante en mesurant l'effet néphélémétrique de celle-ci.

7. Procédé suivant l'une quelconque des revendications 1 à 5, caractérisé en ce que l'on détermine la quantité de particules finement divisées agglutinées ou non agglutinées dans la suspension résultante en mesurant l'effet turbidimétrique de celle-ci.

8. Procédé suivant l'une quelconque des revendications 1 à 5, caractérisé en ce que l'on détermine la quantité de particules finement divisées agglutinées ou non agglutinées dans la suspension résultante en comptant les particules agglutinées ou non agglutinées.

9. Procédé suivant l'une quelconque des revendications 1 à 8, caractérisé en ce que l'on utilise comme particules finement divisées des particules de latex ou des globules rouges.

10. Procédé suivant l'une quelconque des revendications 1 à 9, caractérisé en ce que les anticorps dirigés contre les anticorps IgG sont des anticorps anti-Fc d'IgG et en ce que l'antigène est un lipopolysaccharide provenant de Brucella.

11. Procédé de dosage immunologique d'anticorps IgG + IgA dans un échantillon liquide contenant des anticorps IgM, IgG et IgA, tel qu'un fluide biologique, caractérisé en ce que l'on détermine la quantité d'anticorps IgM dans l'échantillon liquide par le procédé suivant l'une quelconque des revendications 1 à 10, et en ce que l'on soustrait la quantité d'anticorps IgM ainsi obtenue de la quantité d'anticorps totale IgM + IgG + IgA de manière à obtenir la quantité d'anticorps IgG + IgA.

**Claims**

1. A method of immunological analysis of IgM antibodies directed against at least one given antigen in a liquid sample, such as a biological fluid, containing at least IgM and IgG antibodies, the method being characterised in that it comprises reacting the aforementioned liquid sample containing IgM, IgG and, if required, IgA antibodies (directed against the same antigen), with antibodies directed against IgG antibodies and antibodies directed against IgA antibodies in the case where the aforementioned sample contains IgA antibodies, adding to the resulting reaction product the same antigen bonded to finely divided particles and a free antigen of the same kind as the bonded antigen, and determining the degree of agglutination of non-agglutination of the finely divided particles, the quantity of IgM antibodies being inversely proportional to the quantity of non-agglutinated finely-divided particles.

2. A method according to claim 1, characterised in that after the antibodies directed against IgG and, if required, the antibodies directed against IgA have been added, the resulting solution is incubated with agitation at a temperature of about 37 °C for a time of the order of 1 to 15 minutes.

3. A method according to claim 1 or 2, characterised in that after the free antigen and the antigen bonded to finely divided particles have been added as aforementioned, the resulting suspension is incubated with agitation at a temperature of about 37 °C for a time of the order of 1 to 15 minutes.

4. A method according to claim 2 or 3, characterised in that after adding the antibodies directed against IgG and, if required, antibodies directed against IgA and incubating the resulting solution, aggregated IgG are added to the solution to neutralise the effect of the rheumatoid factor.

5. A method according to claim 4, characterised in that after adding the aggregated IgG to the resulting incubated solution, the solution is re-incubated with agitation at a temperature of about 37 °C for a time of the order of 1 to 15 minutes.

6. A method according to any of claims 1 to 5, characterised in that the quantity of agglutinated or non-agglutinated finely-divided particles in the resulting suspension is determined by measuring the nephelometric effect thereof.

7. A method according to any of claims 1 to 5, characterised in that the quantity of agglutinated or non-agglutinated finely-divided particles in the resulting suspension is determined by measuring the turbidimetric effect thereof.

8. A method according to any of claims 1 to 5, characterised in that the quantity of agglutinated or non-agglutinated finely-divided particles in the resulting suspension is determined by counting the agglutinated or non-agglutinated particles.

9. A method according to any of claims 1 to 8, characterised in that latex particles or erythrocytes are used as the finely-divided particles.

10. A method according to any of claims 1 to 9, characterised in that the antibodies directed against IgG antibodies are anti-Fc antibodies of IgG and the antigen is a lipopolysaccharide originating from Brucella.

11. A method of immunological analysis of IgG + IgA antibodies in a liquid sample, such as a biological fluid, containing IgM, IgG and IgA antibodies, characterised in that the quantity of IgM antibody is determined in the liquid sample by the method according to any of claims 1 to 10, and the resulting quantity of IgM antibody is subtracted from the total quantity of IgM + IgG + IgA antibodies so as to obtain the quantity of IgG + IgA antibodies.

**Patentansprüche**

1. Verfahren zur immonologischen Dosierung von IgM-Antikörpern, die gegen wenigstens ein bestimmtes Antigen gerichtet sind, in eine flüssige Probe, die wenigstens IgM- und IgG-Antikörper enthält, wie beispielsweise eine biologische Flüssigkeit, dadurch gekennzeichnet, daß die genannte flüssige Probe, die die IgM-, IgG- und ggf. IgA-Antikörper enthält (die gegen dasselbe Antigen gerichtet sind) mit gegen die IgG gerichteten Antikörper und gegen die IgA gerichteten Antikörper, im Falle daß die genannte Probe die IgA-Antikörper enthält, in Reaktion versetzt werden, zu dem so erthaltenen Reaktionsprodukt das gleiche Antigen, daß an fein verteilte Partikel gebunden ist, und ein freies Antigen desselben Typs, wie das gebundene Antigen, hinzugefügt wird und das Ausmaß der Agglutination oder der Nicht-Agglutination dieser fein verteilten Partikel bestimmt wird, wobei die Menge der IgM-Antikörper umgekehrt proportional zur Menge der nicht-agglutinierten fein verteilten Partikel ist.

2. Verfahren nach Anspruch 1, dadurch gekennzeichnet, daß nach dem Hinzufügen der gegen die IgG gerichteten Antikörper und ggf. der gegen die IgA gerichteten Antikörper man die so erhaltene Lösung unter Umrühren bei einer Temperatur von ungefähr 37 °C für eine Dauer in der Größenordnung zwischen 1 und 15 min incubiert.

3. Verfahren nach einem der Ansprüche 1 und 2, dadurch gekennzeichnet, daß nach dem Hinzufügen des oben Erwähnten, an fein verteilte Partikel gebundenen Antigens und des freien Antigens man die so erhaltene Lösung unter Umrühren bei einer Temperatur von etwa 37 °C für eine Zeitdauer in der Größenordnung von 1 bis 15 min incubiert.

4. Verfahren nach einem der Ansprüche 2 und 3, dadurch gekennzeichnet, daß nach dem Hinzufügen der gegen die IgG gerichteten Antikörper und ggf. der gegen die IgA gerichteten Antikörper und dem Incubieren der so erhaltenen Lösung man zu dieser angehäufte IgG hinzufügt, um den durch den Rhumatoid-Faktor hervorgerufenen Effekt zu neutralisieren.

5. Verfahren nach Anspruch 4, dadurch gekennzeichnet, daß nach dem Hinzufügen der angehäuften IgG zur so erhaltenen incubierten Lösung man diese unter Umrühren bei einer Temperatur von etwa 37 °C für eine Zeitdauer in der Größenordnung von 1 bis 15 min erneut incubiert.

6. Verfahren nach einem der Ansprüche 1 bis 5, dadurch gekennzeichnet, daß man die Menge der in der erhaltenen Suspension agglutinierten oder nicht-agglutinierten fein verteilten Partikel bestimmt, indem man den Trübungseffekt derselben mißt.

7. Verfahren nach einem der Ansprüche 1 bis 5, dadurch gekennzeichnet, dass man die Menge der in der erhaltenen Suspension .agglutinierten oder nicht-agglutinierten fein verteilten Partikel bestimmt, indem man den Trübungseffekt derselben misst.

8. Verfahren nach einem der Ansprüche 1 bis 5, dadurch gekennzeichnet, daß man die Menge der in der erhaltenen Suspension agglutinierten oder nicht-agglutinierten fein verteilten Partikel bestimmt, indem man die agglutinierten oder nicht-agglutinierten Partikel zählt.

9. Verfahren nach einem der Ansprüche 1 bis 8, dadurch gekennzeichnet, daß man als fein verteilte Partikel Latex-partikel oder rote Blutkörperchen verwendet.

10. Verfahren nach einem der Ansprüche 1 bis 9, dadurch gekennzeichnet, daß gegen die IgG-Antikörper gerichteten Antikörper Anti-Fc-Antikörper von IgG sind und daß das Antigen ein von Brucella stammendes Lipopolysaccharid ist.

11. Verfahren zur immunologischen Dosierung von IgG + IgA-Antikörpern in eine flüssige Probe, die IgM-, IgG- und IgA-Antikörper enthält, wie beispielsweise eine biologische Flüssigkeit, dadurch gekennzeichnet, daß man die Menge der IgM-Antikörper in der flüssigen Probe durch das Verfahren nach einem der Ansprüche 1 bis 10 bestimmt, und daß man die so erhaltene IgM-Antikörpermenge von der Gesamtmenge der IgM + IgG + IgA-Antikörper abzieht, um die Menge der IgG + IgA-Antikörper zu erhalten.

Fig.1.

Fig.2.

NOMBRE DE PARTICULES LIBRES
EN UNITES ARBITRAIRES (U.A.)

ECHANTILLON 1/25 DE 50 µl

1 / DILUTION DU SERUM

EP 0 189 389 B1

Ac IgM   anti BRUCELLA

Fig.3.

EP 0 189 389 B1

# Ac Ig M TOXOPLASMA

Fig.4.